Europäisches Patentamt

European Patent Office

Office européen des brevets

(19)

(11) Publication number: **0 375 875**
**A2**

(12) **EUROPEAN PATENT APPLICATION**

(21) Application number: **89119827.7**

(22) Date of filing: **24.11.89**

(51) Int. Cl.⁵: **C12N 15/52, A01H 1/00, A01N 25/00, A01N 43/00, A01N 47/00**

(30) Priority: **30.12.88 US 292207**

(43) Date of publication of application:
**04.07.90 Bulletin 90/27**

(84) Designated Contracting States:
**AT BE CH DE ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Newhouse, Keith Elling**
**3212 Leeland Drive**
**Bensalem Pennsylvania 19020(US)**
Inventor: **Schaefer, Thomas Joseph**
**2427 Village of Pennbrook**
**Levittown Pennsylvania 19054(US)**

(74) Representative: **Wächtershäuser, Günter, Dr.**
**Tal 29**
**D-8000 München 2(DE)**

(54) **A method to improve the protection of crops from herbicidal injury.**

(57) The present invention provides a method for protecting a crop from herbicidal injury by incorporating genetic resistance into the crop in combination with treating seed of the crop with a chemical safener.

EP 0 375 875 A2

# A METHOD TO IMPROVE THE PROTECTION OF CROPS FROM HERBICIDAL INJURY

The invention herein described relates to a method to provide improved safety to agronomically important plants by the combination of the use of genetically imparted resistance to said plants with the use of a chemical antidote applied either to the seed of said plants or mixed with the herbicide prior to application.

Now it has been discovered that the combination of introduced genetic herbicide resistance with the use of a chemical safener provides significantly enhanced protection in those cases where said genetic resistance or use of a chemical safener alone do not provide sufficient protection.

The present invention is directed to a method for protecting a crop from inhibition of growth and/or development due to a herbicidal compound by incorporating genetically imparted resistance to the herbicide in combination with treating seed of said crop with an antidotal amount of a chemical safener to the herbicide.

The genetically imparted resistance may be present within the crop species, introduced from related species, selected by tissue culture, developed by mutation breeding methods, or introduced by other gene transfer methods including, but not limited to, those involving genetic engineering and plant transformation techniques. The chemical safener may be applied to the seed of said crop or introduced into the seed zone by alternative methods including, but not limited to, spraying and granular application. Alternatively, the chemical safener can be applied as a broadcast spray to the soil and/or to the foliage of an emerged crop. The chemical safener can be applied separately, or combined with the herbicide, prior to application.

Methods for the production of plants, plant tissue and plant seed which contain a resistant acetohydroxyacid synthase (AHAS) enzyme are well known in the art. Plants, plant tissue and plant seed bred to include a gene encoding a resistant AHAS enzyme demonstrate resistance to growth inhibition by a herbicide at concentrations which normally inhibit the growth and development of said plants, plant tissue and plant seed not possessing a resistant form of AHAS.

## BRIEF DESCRIPTION OF THE DRAWING

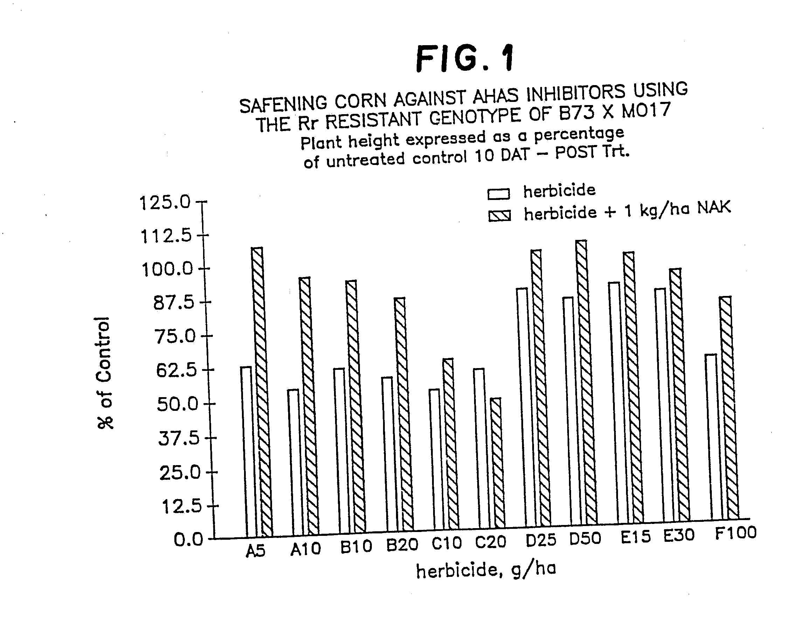

Fig. 1 is a graph of the combined effect of a chemical safener applied post-emergence and heterozygous resistant hybrid seed for increased crop protection against AHAS inhibiting herbicides.

## DETAILED DESCRIPTION OF THE INVENTION

An embodiment of the invention is described wherein a herbicidal class is capable of inhibiting the AHAS enzyme in plants. Such herbicides include sulfonylureas and imidazolinones and are advantageous because they may be used at relatively low rates of application while maintaining effective control of a broad spectrum of weed species. It has now been found that crops can be protected from damage caused by this class of herbicides by incorporating a resistance gene in a heterozygous state in the crops to the herbicide in combination with treating the seed of the crops with naphthalic anhydride or a water soluble salt of naphthalic acid. The safener can be applied either as a seed treatment or as a spray applied pre-plant, pre-emergence or post-emergence to the crop.

An embodiment of this invention is herein-below described wherein the herbicidal agents are compounds which inhibit the AHAS enzyme in plants, such as certain sulfonyl ureas and imidazolinones, the imparted genetic resistance is the incorporation of a resistance gene in a heterozygous state in said plants, and the chemical safener includes 1,8-naphthalic anhydride and the dispotassium salt of naphthalic acid, applied either alone or in combination as a seed treatment or as a tank mixed spray either pre-plant, pre-emergence to the soil or post-emergence to said plants.

In this embodiment corn tissue cultures which are resistant to an AHAS inhibiting herbicide are selected, and plants which are resistant to the herbicide are regenerated from these cultures. These plants are cross pollinated with plants of an herbicide sensitive inbred line. Seeds obtained from the mature plants resulting from these crossings are planted, grown to sexual maturity and self-pollinated. The selfing process is repeated for a second generation. Seed is harvested from individual plants and kept separate. Homozygous resistant progeny can be identified from these plants by using a seedling assay for herbicide resistance.

The herbicide resistance gene can be introgressed by standard breeding methods to create commercial cultivars homozygous for the resistance trait. In the case wherein the plant is corn, these cultivars will be inbred lines uniformly homozygous for the resistance trait. These homozygous resistant inbred lines are then used, as either a male or female parent, in crosses with plants lacking the resistance gene to produce hybrid seed which is uniformly herbicide resistant and heterozygous for the resistance gene.

Compounds which are effective herbicides due to their ability to inhibit the growth and development of plants by inhibiting the AHAS enzyme can be selected from imidazolinones such as 5-ethyl-2-(4-isopropyl-4-methyl-5-oxo-2-imidazolin-2-yl)nicotinic acid, 5-methyl-2-(4-isopropyl-4-methyl-5-oxo-2-imidazolin-2-yl)-nicotinic acid, 2-(4-isopropyl-4-methyl-5-oxo-2-imidazolin-2-yl)nicotinic acid, methyl-2-(4-isopropyl -4-methyl-5-oxo-2-imidazolin-2-yl)m-and p-toluate and 2-(4-isopropyl-4-methyl-5-oxo-2-imidazolin-2-yl)-3-quinolinecarboxcylic acid; and sulfonylureas such as methyl-o-{[(4-methoxy-6-methyl-s-triazin-2-yl)carbamoyl]-sulfamoyl}benzoate, ethyl-o-{[(4-chloro-6-methoxy-2-pyrimidinyl)carbamoyl]sulfamoyl}benzoate, 1-[(o-chlorophenyl)sulfonyl]-3(4-methoxy-6-methyl-s-triazin-2-yl)urea, methyl -3-{[(4-methoxy-6-methyl-s-triazin-2-yl)carbamoyl]sulfamoyl}-2-thiophenecarboxylate and methyl-o-{[3-(4,6-dimethylpyrimidin-2-yl)ureido]-sulfonyl}benzoate.

for improved crop protection against damage caused by pre-emergence soil applications of AHAS inhibiting herbicides or injury caused by follow crop residues of AHAS inhibiting herbicides already present in the soil, the heterozygous resistant hybrid seed is uniformly coated with 1,8-naphthalic anhydride as a 20% to 40% wettable powder formulation, preferably 20%, by mixing together 1.0 g of formulated 1,8-naphthalic anhydride and 100 g of heterozygous resistant seed. The coated seed is then planted according to agronomic conventions and the herbicide is applied to the soil before or after planting, but before crop plants emerge.

For increased crop safety against damage cuased by post-emergence foliar applications of AHAS inhibiting herbicides, the heterozygous seed, coated or uncoated with 1,8-naphthalic anhydride, is planted and allowed to grow until the second to early third leaf stage. The plants are then sprayed with an aqueous solution of the dipotassium salt of 1,8-naphthalic acid at a rate of about 0.35 to 1.0 kg/ha, preferably about 0.50 to 0.75 kg/ha, pre-mixed with a herbicide. The herbicide is preferably applied at a rate of about 0.005 to 0.500 kg/ha.

In order to facilitate a further understanding of the invention, the following examples are presented primarily for the purpose of illustrating more specific details thereof. Unless otherwise noted, all parts are by weight.

## EXAMPLE 1

Evaluation of the combination of genetic resistance plus a chemical safener for crop protection against post-emergence application of an AHAS inhibiting herbicide

The corn seeds used in this experiment are in open pedigreed hydrid (B73xMO17) designated as rr, and the same hybrid containing a single resistance gene (XA17), designated as Rr, the chemical safener is the dipotassium salt of 1,8-naphthalic acid (NAK), and the herbicide is 5-ethyl-2-(4-isopropyl-4-methyl-5-oxo-2-imidazolin-2-yl)nicotinic acid. The seeds are planted in six inch azalea pots with a BACCTO® Professional Planting Mix containing sphagnum peat moss, vermiculite, perlite, limestone, superphosphate (0-46-0), calcium nitrate, potassium nitrate, and complete trace elements manufactured by Michigan Peat Company, using either two seeds (Rr) or four seeds (rr) per pot. After emergence, the plants are thinned to two plants per pot. At the 3-4 leaf stage, the plants are sprayed with aqueous solutions containing 0-0.25% NAK, 0-0.0175% herbicide and 0.5% TWEEN-20®, a polyoxyethylene sorbitan monolaurate surfactant of Atlas Chemical Industries, at such a rate as to provide from 0.00 to 1.00 kg/ha of NAK and from 0.00 to 0.07 kg/ha of an herbicide. Each individual treatment is replicated four times. Before applying treatments, plant heights are recorded; after treatment, the plants are placed on greenhouse benches and cared for in the usual manner commensurate with conventional greenhouse practices. Plant heights are measured and recorded at 0, 7, 12, 17 and 21 days after treatment (DAT). Mean heights are determined from these data. The results are shown in Table I.

TABLE 1

| Improved Protection Of Corn From The Inhibition Of Growth And Development Caused By An AHAS Inhibiting Herbicide | | | | | | | | | | | | |
|---|---|---|---|---|---|---|---|---|---|---|---|---|
| Mean plant heights, cm | | | | | | | | | | | | |
| Herbicide (kg/ha) | Hybrid | NAK (kg/ha) | 0 DAT (cm) | (% of Control) | 7 DAT (cm) | (% of Control) | 12 DAT (cm) | (% of Control) | 17 DAT (cm) | (% of Control) | 21 DAT (cm) | (% of Control) |
| 0.00 | rr | 0.00 | 26 | - | 50 | - | 66 | - | 78 | - | 86 | - |
| 0.00 | Rr | 0.00 | 29 | - | 51 | - | 70 | - | 80 | - | 86 | - |
| 0.0175 | rr | 0.00 | 28 | (107) | 33 | (67) | 36 | (54) | 37 | (48) | 41 | (49) |
| 0.0175 | rr | 1.00 | 28 | (107) | 33 | (66) | 52 | (72) | 69 | (88) | 76 | (90) |
| 0.0175 | Rr | 0.00 | 28 | (96) | 39 | (72) | 60 | (84) | 72 | (89) | 80 | (93) |
| 0.0175 | Rr | 1.0 | 27 | (93) | 44 | (81) | 60 | (84) | 70 | (86) | 76 | (83) |
| 0.070 | rr | 0.0 | 26 | (100) | 30 | (57) | 30 | (46) | 0 | (0) | 0 | (0) |
| 0.070 | rr | 1.0 | 27 | (104) | 31 | (61) | 37 | (56) | 50 | (64) | 58 | (68) |
| 0.070 | Rr | 0.0 | 30 | (104) | 35 | (65) | 41 | (57) | 50 | (63) | 62 | (52) |
| 0.070 | Rr | 1.0 | 29 | (100) | 45 | (83) | 65 | (92) | 75 | (94) | 82 | (95) |

EXAMPLE 2

Evaluation of the effectiveness of the combination of heterozygous resistant corn and a chemical safener, applied as a seed coating and as a tank mix, for crop protection against pre-emergence applications of AHAS inhibiting herbicides

The corn seed used in this experiment is a heterozygous resistant hybrid (B73xMO17) containing a single copy of a resistance gene (XA17). A portion of the seed is coated by mixing 100 g of seed with 1.0 g of a 20% wettable powder formulation of 1,8-naphthalic anhydride (NA). Said formulation consisting of 4% sodium lignin sulfonate, 0.8% sodium N-methyl-N-oleoyl-taurate, 75.2% magnesium aluminum silicate, and 20% 1,8-naphthalic anhydride. The seed and the formulated 1,8-naphthalic anhydride are placed in a bag and shaken until all the seed is uniformly coated. The coated and uncoated seeds are planted in five inch azalea pots with sterilized Princeton greenhouse soil using two seeds per pot. After planting, the pots are sprayed with an aqueous solution containing 0-0.19% of the dipotassium salt of naphthalic acid (NAK), 0-0.0625% of an herbicide, and 0.50% TWEEN-20®, at such a rate as to provide from 0-750 g/ha of NAK and from 0-250 g/ha of an herbicide. Each individual treatment is replicated three times. After spraying, the pots are placed on greenhouse benches and cared for in accordance with conventional greenhouse procedures. Plant heights are measured at 20 days after treatment (DAT). Mean heights are determined from these data. The results are shown in Table II.

TABLE II

| Improved Protection of Heterozygous Resistant Corn From Pre-Emergence Applications of AHAS Inhibiting Herbicides | | | | | |
|---|---|---|---|---|---|
| Mean Plant Heights, cm | | | | | |
| Herbicide | Rate g/ha | NA *Seed coating | NAK Tank Mix g/ha | 20 DAT cm | % Control |
| | 0 | - | 0 | 52.2 | - |
| | 0 | - | 750 | 48.3 | (93) |
| 5-Ethyl-2-(4-isopropyl-4-methyl-5-oxo-2-imidazolin-2-yl)nicotinic acid[a] | 32 | - | 0 | 46.2 | (89) |
| | 32 | - | 750 | 50.3 | (96) |
| | 32 | + | 0 | 47.2 | (90) |
| | 63 | - | 0 | 38.2 | (73) |
| | 63 | - | 750 | 50.7 | (97) |
| | 63 | + | 0 | 48.0 | (92) |
| | 125 | - | 0 | 41.3 | (79) |
| | 125 | - | 750 | 46.3 | (89) |
| | 125 | + | 0 | 46.0 | (88) |
| | 250 | - | 0 | 29.0 | (56) |
| | 250 | - | 750 | 38.5 | (74) |
| | 250 | + | 0 | 38.0 | (73) |
| 5-Methyl-2-(4-isopropyl-4-methyl-5-oxo-2-imidazolin-2-yl)nicotinic acid[b] | 63 | - | 0 | 42.0 | (80) |
| | 63 | - | 750 | 45.8 | (88) |
| | 63 | + | 0 | 50.5 | (97) |
| | 250 | - | 0 | 27.8 | (53) |
| | 250 | - | 750 | 34.2 | (66) |
| | 250 | + | 0 | 42.2 | (81) |

*A seed coating treatment is designated by ( + ).
[a]Expected use rate is from 50 g/ha to 100 g/ha.
[b]Expected use rate is from 30 g/ha to 80 g/ha.

EXAMPLE 3

Evaluation of the effectiveness of the use of a chemical safener on heterozygous resistant corn for increased crop protection against post-emergence applications of an AHAS inhibiting herbicide

The corn seed used in this experiment is a heterozygous resistant hybrid (B73xMO17) containing a single copy of a resistance gene (XA17). A portion of the seed is coated by mixing 100 g of seed with 1.0 g of a 20% wettable powder formulation of 1,8-naphthalic anhydride (NA) as described in Example 2. The coated and uncoated seeds are planted in six inch azalea pots with a BACCTO® Professional Plant Mix using three seeds per pot. AT the three leaf stage, the plants are thinned to two uniform plants per pot and are sprayed with aqueous solutions containing 0.00-0.19% of the potassium salt of 1,8-naphthalic anhydride (NAK), 0-0.0175% herbicide, and 0.5% TWEEN-20® at such a rate as to provide from 0 to 750 g/ha of NAK and from 0 to 62.5 g/ha of an herbicide. Each individual treatment is replicated three times. After spraying, the pots are placed on greenhouse benches and cared for in accordance with conventional greenhouse procedures. Plant heights are measured and recorded at ten days after treatment (DAT). Mean heights are determined from these data. The results are shown in Table III.

5

TABLE III

| Improved Protection of Heterozygous Resistant Corn From Post-Emergence Applications of AHAS Inhibiting Herbicides | | | | | |
|---|---|---|---|---|---|
| Mean Plant Heights, cm | | | | | |
| Herbicide | Rate g/ha | Na Seed coating | NAK Tank Mix g/ha | 10 DAT cm | % Control |
| | 0 | - | 0 | 63.5 | - |
| | - | 0 | 750 | 64.8 | (102) |
| 5-Ethyl-2-(4-isopropyl-4-methyl-5-oxo-2-imidazolin-2-yl)nicotinic acid[a] | 32 | - | 0 | 46.5 | (73) |
| | 32 | - | 750 | 58.5 | (92) |
| | 63 | - | 0 | 46.3 | (73) |
| | 63 | - | 750 | 58.8 | (93) |
| | 63 | + | 0 | 50.3 | (79) |
| 5-Methyl-2-(4-isopropyl-4-methyl-5-oxo-2-imidazolin-2-yl)nicotinic acid[b] | 32 | - | 0 | 42.5 | (67) |
| | 32 | - | 750 | 59.5 | (94) |
| | 32 | + | 0 | 48.6 | (77) |

[a]Expected use rate is from 50 g/ha to 100 g/ha.
[b]Expected use rate is from 30 g/ha to 80 g/ha.

## EXAMPLE 4

Evaluation of the combined effect of a chemical safener applied post-emergence and heterozygous resistant hybride seed increased crop protection against AHAS inhibiting herbicides

The heterozygous resistant, hybrid corn seed, is planted in six inch azalea pots using two seeds per pot with a BACCTO® Professional Plant Mix which has been supplemented with OSMOCOTE®, a controlled release fertilizer containing 18% nitrogen, 6% phosphoric acid, and 27% potash manufactured by the Sierra Chemical Company. The seeds are allowed to germinate and grow until the three leaf stage, and are then sprayed with an aqueous solution containing 0-0.25% of the dipotassium salt of 1,8-naphthalic acid (NAK), 0-0.0175% of an herbicide and 0.5% TWEEN-20® at such a rate as to provide from 0-1,000 g/ha NAK and from 0-100 g/ha of a herbicide. Each treatment is replicated three times. The pots are placed on greenhouse benches and are cared for in the usual manner commensurate with conventional greenhouse practices. Plant heights are measured at ten days after treatment (DAT). Mean plant heights are determined from these data. The results are shown in Table IV and are graphically illustrated in Figure 1.

## TABLE IV

### Safening Corn Against AHAS Inhibitors Using
### Genetic Resistance And A Chemical Antidote
### Mean Plant Heights, cm

| | Herbicide | Rate g/ha | NAK g/ha | 10 DAT cm | % Control |
|---|---|---|---|---|---|
| | | 0.0 | 0.0 | 56 | - |
| A | Methyl-o-{[(4 -methoxy-6-methyl -s-triazin-2-yl)- carbamoyl]sul- famoyl}benzoate | 5.0 | 0.0 | 35 | 63 |
| | | 5.0 | 1000.0 | 60 | 107 |
| | | 10.0 | 0.0 | 30 | 54 |
| | | 10.0 | 1000.0 | 53 | 95 |
| B | Methyl-o-{[3-(4,6- dimethylpyrimidin -2-yl)ureido]- sulfonyl}benzoate | 10.0 | 0.0 | 29 | 52 |
| | | 10.0 | 1000.0 | 35 | 63 |
| | | 20.0 | 0.0 | 33 | 59 |
| | | 20.0 | 1000.0 | 27 | 48 |
| C | Methyl-3-{[(4 -methoxy-6-methyl -s-triazin-2-yl)- carbamoyl]sul- famoyl}-2-thio- phenecarboxylate | 25.0 | 0.0 | 49 | 88 |
| | | 25.0 | 1000.0 | 57 | 102 |
| | | 50.0 | 0.0 | 47 | 84 |
| | | 50.0 | 1000.0 | 59 | 105 |

7

## TABLE IV, Continued

| | Herbicide | Rate g/ha | NAK g/ha | 10 DAT cm | % Control |
|---|---|---|---|---|---|
| | Ethyl-o-{[(4-Chloro | 15.0 | 0.0 | 50 | 89 |
| | -6-methoxy-2-pyri- | 15.0 | 1000.0 | 56 | 100 |
| D | midinyl)carbamoyl]- | 30.0 | 0.0 | 48 | 86 |
| | sulfamoyl}benzoate | 30.0 | 1000.0 | 52 | 93 |
| | 1-[(o-Chlorophenyl)- | 10.0 | 0.0 | 34 | 61 |
| | sulfonyl]-3-(4- | 10.0 | 1000.0 | 52 | 93 |
| E | methoxy-6-methyl-s | 20.0 | 0.0 | 32 | 57 |
| | -triazin-2-yl)urea | 20.0 | 1000.0 | 48 | 86 |
| | 5-Ethyl-2-(4-iso- | | | | |
| | propyl-4-methyl-5 | 100.0 | 0.0 | 34 | 61 |
| F | -oxo-2-imidazolin | 100.0 | 1000.0 | 46 | 82 |
| | -2-yl)nicotinic | | | | |
| | acid | | | | |

Claims

1. A method for protecting a crop from inhibition of growth and/or development due to a herbicidal compound which comprises incorporating genetically imparted resistance to said crop to the herbicide in combination with treating seed of said crop with a chemical safener to the herbicide.

2. The method according to claim 1 wherein the crop is a broadleaf crop selected from the group consisting of alfalfa, clover, cotton, cucumber, edible beans, edible peas, lentil, melons, peanut, potato, rapeseed, safflower, sesame, soybean, sugarbeet, sunflower tobacco, and tomato.

3. The method according to claim 1 wherein the crop is a cereal crop selected from the group consisting of barley, corn, oats, millet, rice, rye, sorghum and wheat.

4. The method according to claim 1 wherein the herbicidal compound is an inhibitor of an acetohydroxyacid synthase enzyme and the resistance is imparted by a gene encoding for an acetohydroxyacid synthase enzyme which is resistant acetohydroxyacid synthase inhibiting herbicides.

5. The method according to claim 4 wherein the AHAS inhibiting herbicidal compound is an imidazolinone selected from the group consisting of 5-ethyl-2-(4-isopropyl-4-methyl-5-oxo -2-imidazolin-2-yl)-nicotinic acid, 5-methyl-2-(4-iso-propyl-4-methyl-5-oxo-2- imidazolin-2-yl)nicotinic acid, 2-(4-isopropyl-4-methyl-5-oxo-2-imidazolin-2-yl)-3-quinolinecarboxylic acid, 2-(4-isopropyl-4-methyl-5-oxo-2-imidazolin-2-yl)-nicotinic acid, and methyl-2-(4-isopropyl-4-methyl-5-oxo-2-imidazolin-2-yl)-m-and p-toluate.

6. The method according to claim 4 wherein the AHAS inhibiting herbicidal compound is a sulfonyl urea selected from the group consisting of methyl-o-{[(4-methoxy-6-methyl-s-triazin -2-yl)carbamoyl] sulfamoyl}benzoate, ethyl-o-{[(4-chloro-6-methoxy-2-pyrimidinyl)carbamoyl]sulfamoyl}benzoate, 1-[(o-chlorophenyl)sulfonyl]-3-(4-methoxy-6-methyl-s-triazin-2-yl)urea, methyl-3-{[4-methoxy-6-methyl-s-triazin-2-yl)carbamoyl]sulfamoyl}-2-thiophenecarboxylate and methyl-o-{[3-(4,6-di-methylpyrimidin-2-yl)ureido]-sulfonyl}benzoate.

8

7. The method according to claim 4 wherein the gene is XA17 which is present in the heterozygous condition.

8. The method according to claim 1 wherein the chemical safener is 1,8-naphthalic anhydride and the dicationic salt of 1,8-naphthalic acid.

9. The method according to claim 8 wherein the dicationic salt of 1,8-naphthalic acid is selected from the group consisting of alkali metals, alkaline earth metals, ammonium or organic ammonium comprised of a positively charged nitrogen atom joined to form one to four alphatic groups, each containing 1 to 6 carbon atoms.

10. The method according to claim 9 wherein the dicationic salt of 1,8-naphthalic acid is the dipotassium salt of 1,8-naphthalic acid.

# FIG. 1

## SAFENING CORN AGAINST AHAS INHIBITORS USING THE Rr RESISTANT GENOTYPE OF B73 X MO17

Plant height expressed as a percentage
of untreated control 10 DAT — POST Trt.

Legend:
- □ herbicide
- ▨ herbicide + 1 kg/ha NAK

Y-axis: % of Control (0.0, 12.5, 25.0, 37.5, 50.0, 62.5, 75.0, 87.5, 100.0, 112.5, 125.0)

X-axis: herbicide, g/ha (A5, A10, B10, B20, C10, C20, D25, D50, E15, E30, F100)

EP 0 375 875 A2